# EUROPEAN PATENT APPLICATION

(11) **EP 3 404 018 A1**
(43) Date of publication of application: **21.11.2018**
(21) Application number: 17171060.1
(22) Date of filing: 15.05.2017
(51) Int. Cl.: C07C 319/28, C07C 323/58

(54) **PROCESS FOR PREPARING METHIONINE**

(71) Applicant: Evonik Degussa GmbH, 45128 Essen (DE)
(72) Inventor: BERNHARDT, Sebastian, 63739 Aschaffenburg (DE); KÖRFER, Martin, 63796 Kahl (DE); REICHERT, Stefan, 60438 Frankfurt (DE); HASSELBACH, Hans Joachim, 63571 Gelnhausen (DE); DRAPAL, Bernd, 63755 Alzenau (DE); PETER, Rainer, 63829 Krombach (DE); KAISER, Christian, 63857 Waldaschaff (DE); HARTONO, Benny, 119916 Singapore (SG); JAKOB, Harald, 63594 Hasselroth (DE)

(57) **Abstract**

The present invention relates to a process for preparing methionine, comprising the alkaline hydrolysis of 5-(2-methylmercaptoethyl)-hydantoin (methionine hydantoin) in an aqueous process solution comprising alkali metal hydroxide and/or alkali metal carbonate and/or alkali metal hydrogencarbonate wherein the alkali metal cations in the process solution are potassium and sodium having a K/Na molar ratio range from 99/1 to 20/80.

## Description

The present invention relates to a process for preparing methionine, comprising the alkaline hydrolysis of 5-(2-methylmercaptoethyl)-hydantoin (methionine hydantoin) in an aqueous process solution comprising alkali metal hydroxide and/or alkali metal carbonate and/or alkali metal hydrogencarbonate wherein the alkali metal cations in the process solution are potassium and sodium having a K/Na molar ratio range from 99/1 to 20/80.

The amino acid methionine is currently industrially produced worldwide in large amounts and is of considerable commercial importance. Methionine is employed in many fields, such as pharmaceutical, health and fitness products, but particularly as feedstuff additive in many feedstuffs for various livestock.

On an industrial scale, methionine is produced chemically via the Bucherer-Bergs reaction, which is a variant of the Strecker synthesis. Here, the starting substances 3-methylmercaptopropanal (prepared from 2-propenal and methylmercaptan), hydrocyanic acid (hydrogen cyanide), ammonia and carbon dioxide are reacted to give 5-(2-methylmercaptoethyl)hydantoin (methionine hydantoin) and this is subsequently hydrolysed by alkali with potassium carbonate and potassium hydrogen carbonate to give potassium methioninate. Methionine is finally liberated from its potassium salt by treatment with carbon dioxide ("carbonation reaction"), which may be filtered off as a precipitate from the mother liquor containing potassium carbonate and potassium hydrogen carbonate (US 5,770,769). The precipitated methionine is then recrystallized from an aqueous solution, filtered off and dried. The methionine so obtained has a purity greater than 99 % and a potassium content of less than 0.5 %.

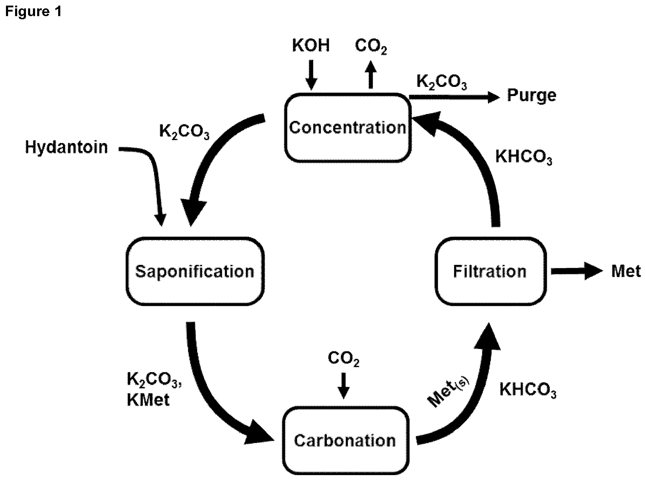

The ammonia, potassium carbonate and potassium hydrogen carbonate reagents and also carbon dioxide are generally recycled in industrial methionine production. However, it is necessary to continuously exchange a part of the aqueous process solution of this hydantoin hydrolysis circulation for fresh potassium hydroxide, essentially to remove ("purge") inactivated potassium salt from the circulation in the form of potassium formate. Potassium formate forms from the residues of hydrogen cyanide being present in the methionine hydantoin solution and alkaline potassium salts from the hydantoin hydrolysis (WO2013030068A2). A further by-product of the methionine synthesis is the dipeptide methionylmethionine (EP 1 564 208 A1). In general, the excessive enrichment of by-products in the hydantoin hydrolysis circulation must be avoided since otherwise disruptions in crystal formation occur downstream. A scheme of the hydantoin hydrolysis circulation is shown in figure 1.

A typical process solution recycled to the hydantoin hydrolysis circulation comprises approximately 10 to 16 % by weight potassium, 5 to 10 % by weight methionine, 3 to 5 % by weight methionylmethionine, 0.5 to 2.0 % by weight formate, and 0.2 to 0.3 % by weight acetate.

Methionate and potassium may be partially recovered from the purge stream by precipitation with carbon dioxide in form of methionine and potassium carbonate, respectively, filtered off and fed back to the process solution. However, there is still a considerable loss of the relatively expensive potassium salts. The final purge solution comprises approximately 2 to 6 % by weight methionine, 4 to 8 % by weight methionylmethionine and 6 to 14 % by weight potassium in the form of potassium salts, 1 to 1.7 % by weight formate, and 0.3 to 0.5 % by weight acetate.

With the aim to recycle the excess of sodium carbonate Capelle and Rey (WO 2016/170252 A1) propose a method for producing methionine by alkaline hydrolysis of methionine hydantoin in aqueous phase, eliminating ammonia and carbon dioxide from the hydrolysis medium, and neutralising the methionine sodium salt obtained, wherein after the elimination of ammonia and carbon dioxide the reaction medium is concentrated in order to precipitate sodium carbonate which is filtered off and recycled for alkaline hydrolysis in the presence of sodium hydroxide and sodium carbonate. However, in this method the neutralisation of the methionine sodium salt is achieved with sulphurous acid still leading to large amounts of sodium sulphate as side product which has to be removed from the process.

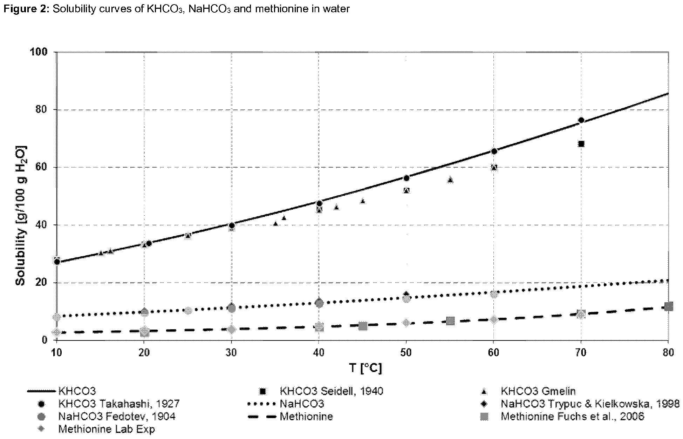

In the carbonation reaction, i.e. reaction of the alkali metal salt of methionine with carbon dioxide to form methionine and alkali metal hydrogencarbonate (Figure 1), methionine is precipitated and removed from the process solution. However, due to the low difference in the solubility of sodium hydrogencarbonate and methionine (Figure 2), large quantities of sodium hydrogencarbonate are co-precipitated with methionine in case that sodium salt of methionine is used in this reaction. Therefore, in the carbonisation reaction the potassium salt of methionine is used, meaning that potassium hydroxide usually is added to the hydantoin hydrolysis circulation process.

The object of the present invention is to provide a process for preparing methionine, comprising the alkaline hydrolysis of 5-(2-methylmercaptoethyl)-hydantoin (methionine hydantoin) in an aqueous process solution comprising potassium hydroxide and/or carbonate and/or hydrogencarbonate to form methionine potassium salt, precipitation of methionine by means of carbon dioxide, separation of the precipitated methionine from the process solution, concentration of the process solution and recycling of the process solution to the alkaline hydrolysis of methionine hydantoin, wherein the potassium cations in the process solution are partly replaced by sodium cations in order to avoid losses of the relatively expensive potassium salts in the purge solution stream.

It was found that in the process solution resulting from the hydantoin hydrolysis reaction a large part of potassium can be replaced by sodium without the undesired co-precipitation of sodium hydrogencarbonate in the carbonation reaction.

This object is achieved by a process for preparing methionine, comprising the alkaline hydrolysis of 5-(2-methylmercaptoethyl)-hydantoin (methionine hydantoin) in an aqueous process solution comprising alkali metal hydroxide and/or alkali metal carbonate and/or alkali metal hydrogencarbonate to form methionine alkali metal salt, precipitation of methionine by means of carbon dioxide, separation of the precipitated methionine from the process solution, concentration of the process solution and recycling of the process solution in the alkaline hydrolysis of methionine hydantoin once more forwarded to the process solution, wherein the alkali metal cations in the process solution are potassium and sodium having a K/Na molar ratio range from 99/1 to 20/80.

During the concentration step the process solution is re-concentrated to its typical starting concentrations of the hydantoin hydrolysis circulation, i.e. comprising approximately 5 to 16 % by weight alkali metal, 5 to 8 % by weight methionine, 3 to 5 % by weight methionylmethionine, 0.7 to 1.1 % by weight formate, and 0.2 to 0.3 % by weight acetate.

In the process according to the present invention a part of the process solution may continuously be exchanged by fresh alkali metal hydroxide solution.

In the process according to the present invention the K/Na molar ratio in the process solution preferably ranges from 90/10 to 20/80. Ideally, the K/Na molar ratio in the process solution ranges from 70/30 to 50/50.

In the process according to the present invention the precipitated methionine is then recrystallized from an aqueous solution, filtered off and dried. The methionine so obtained has a purity greater than 99 % and a potassium content lower than 0.3 % and a sodium content lower than 0.1 %, in particular the potassium content in the crystalline methionine ranges from 0.05 - 0.3 % and the sodium content ranges from 0.01 - 0.1 %.

### Brief description of the Figures

**Figure 1** shows a scheme of the hydantoin hydrolysis circulation.
**Figure 2** shows the solubility curves of KHCO₃, NaHCO₃ and methionine in water. The measured values stem from the following sources.
   Solubility of KHCO₃:
   Seidell, 1940: Seidel, A.; Solubility Data Ser., 1940.
   Gmelin: Gmelins Handbuch, 22, 1938.
   Takahashi, 1927: Takahashi, G.; Bull. Imp. Seric. Stn. Tokyo 29, 1927, 165.
   Solubility of NaHCO₃:
   Trypuc & Kielkowska, 1998: Trypuc, M.; Kielkowska, U.; J. Chem. Eng. Data 43 (2), 1998, 201-204. Fedotev, 1904: Fedotev, P. P., Z. Phys. Chem. 49, 1904, 168.
   Solubility of Methionine:
   Fuchs et al., 2006: Fuchs et al., 2006, Ind. Eng. Chem. Res., 45, 6578-6584.
      Lab Exp: Results of own laboratory experiments (in accordance to Fuchs et al., 2006).

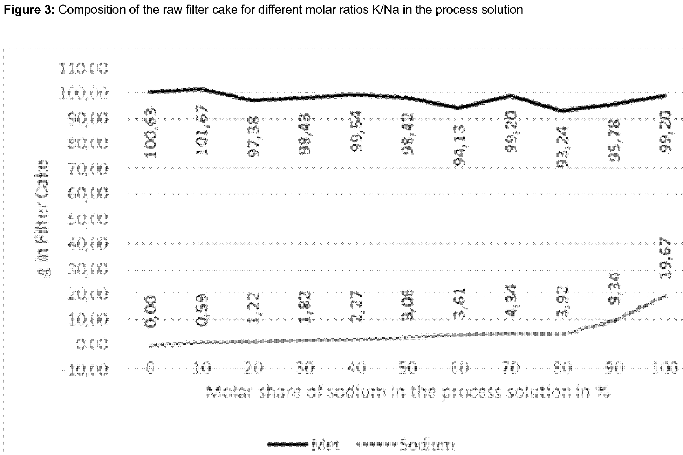

**Figure 3** shows the composition of the raw filter cake for different molar ratios K/Na in the process solution (Example 1).

### Experimental Part

In the carbonation reaction the potassium salt of methionine (K-Met) accompanied by 0.5 equivalents of potassium carbonate is reacted with CO₂. KHCO₃ is formed and methionine (H-Met) is precipitated (Equations 1 and 2).

K-Met + CO₂ + H₂O -> H-Met(s) + KHCO₃ (Equation 1)

K₂CO₃ + CO₂ + H₂O -> 2 KHCO₃ (Equation 2)

Due to the significantly lower solubility of NaHCO₃ compared to KHCO₃ (Figure 2) the molar ratio of K/Na has to be carefully tuned in order to avoid undesired precipitation of NaHCO₃ crystals in the carbonation reactor.

Thus, a series of experiments was carried out in the laboratory to investigate the carbonation reaction with synthetic process solutions for different molar ratios K/Na. The overall alkaline concentration (sum parameter of K and Na) is maintained constant meaning that for a molar ratio K/Na = 70/30 compared to K/Na = 100/0 the potassium concentration is lowered by 30 % and replaced equimolar by sodium. The results of this study are summarized in Figure 3.

The methionine content in the filter cake can be considered being constant for molar ratios K/Na 100/0 to 0/100. The Na-content in the filter cake is slightly increasing for molar ratios K/Na 100/0 to 20/80 up to 3,92 g which can be explained by process solution being attached to and incorporated into the filter cake. However, for molar ratios K/Na 10/90 and 0/100 significantly higher Na content 9.34 - 19.67 g) was observed in the filter cake indicating that NaHCO₃ is also partially precipitated.

Thus, it is possible to run the carbonation reaction up to molar ratios K/Na 20/80 without coprecipitations of sodium hydrogencarbonate.

### Example 1

Experimental procedure for the preparation of a process solution with a molar ratio K/Na of 90/10

A 1000 ml beaker was placed in an ice bath and KOH (40% in water, 354.2 g, 2.53 mol), NaOH (40% in water, 11.2 g, 0.28 mol), D,L-methionine (159.7 g, 1.07 mol), DL-methionyl-DL-methionine (29.9 g, 0.11 mol) and formic acid (50% in water, 41.4 g, 0.45 mol) were dissolved in 200 ml DI-water and stirred for 45 min. The solution was then warmed to 20 °C, transferred to a 1000 ml volumetric flask and filled up with DI-water to 1000 ml total volume.

1100 g of the solution was then transferred to a 2 I Büchi laboratory autoclave and 0.32 g of Defoamer EG2007 were added. Cooling of the autoclave to 30 °C was realized via cooling jacket and cryostat. Mechanical stirring was initiated and the autoclave was pressurized with CO₂ gas to 2 barg. The CO₂ gas feedstream was adjusted to maintain 2 barg until pH 8 was reached and temperature was maintained at 30 °C. Pressure was released from the autoclave and the suspension was transferred to a vacuum filter frit and sucked dry for 15 min at 940 mbar absolute pressure. The filtrate was collected in a 100 ml glas bottle and diluted with DI-water to avoid precipitation of solids afterwards and balanced subsequently. The filter cake was transferred to 3000 ml beaker via spatula and residual solids in the frit were rinsed into the beaker via DI-water. Additional DI-water was added until the solids have been dissolved. The solution was then transferred to a 3 l glass bottle and balanced. Moreover, the residual solids in the autoclave were also rinsed with DI-water collected in a glass bottle as so-called "Holdup" and balanced.

The synthetic process solution, the filter cake dissolved in DI-water, the filtrate and the "Holdup" were analysed for their K and Na content via IPC-OES, Met- & Met-Met via HPLC and formate via ion chromatography and the results are summarized in Table 1.

**Table 1: Carbonation reaction of synthetic process solution with molar ratio K/Na of 90/10.**

| | **Quantity g** | **% Met** | **g Met** | **% Met-Met** | **g Met-Met** | **% K** | **g K** | **% Na** | **g Na** |
|---|---|---|---|---|---|---|---|---|---|
| **Start** | 1099,85 | 14,14 | 155,52 | 2,58 | 28,38 | 8,2 | 90,19 | 0,59 | 6,489 |
| **Filtrate** | 1001,1 | 4,14 | 41,45 | 2,25 | 22,52 | 7,1 | 71,08 | 0,51 | 5,106 |
| **Cake** | 2930,1 | 3,47 | 101,67 | 0,120 | 3,52 | 0,3 | 8,79 | 0,02 | 0,586 |
| **Holdup** | 498,8 | 1,88 | 9,38 | 0,25 | 1,25 | 0,75 | 3,74 | 0,05 | 0,249 |
| **m F+C+H [g]** | | | 152,50 | | 27,29 | | 83,61 | | 5,94 |
| **Accuracy [%]** | | | **98,06** | | **96,16** | | **92,71** | | **91,55** |

## Claims

1. A process for preparing methionine, comprising the alkaline hydrolysis of 5-(2-methylmercaptoethyl)-hydantoin (methionine hydantoin) in an aqueous process solution comprising alkali metal hydroxide and/or alkali metal carbonate and/or alkali metal hydrogencarbonate to form methionine alkali metal salt, precipitation of methionine by means of carbon dioxide, separation of the precipitated methionine from the process solution, concentration of the process solution and recycling of the process solution in the alkaline hydrolysis of methionine hydantoin once more forwarded to the process solution, wherein the alkali metal cations in the process solution are potassium and sodium having a K/Na molar ratio range from 99/1 to 20/80.

2. The process as claimed in claim 1, wherein a part of the process solution is continuously exchanged by fresh alkali metal hydroxide solution.

3. The process as claimed in claim 1 or 2, wherein the K/Na molar ratio in the process solution ranges from 90/10 to 20/80.

4. The process as claimed in claim 3, wherein the K/Na molar ratio in the process solution ranges from 70/30 to 50/50.

5. The process as claimed in any of claims 1 to 4, wherein the precipitated methionine is recrystallized from an aqueous solution, filtered off and dried and wherein the crystalline methionine so obtained has a purity greater than 99 % and a potassium content lower than 0.3 % and a sodium content lower than 0.1 %

6. The process as claimed in claim 5, wherein the potassium content in the crystalline methionine ranges from 0.05 - 0.3 % and the sodium content ranges from 0.01 - 0.1 %.
